# EUROPEAN PATENT SPECIFICATION

(11) **EP 0 700 266 B1**
(45) Date of publication and mention of the grant of the patent: **02.01.2003**
(21) Application number: 93910730.6
(22) Date of filing: 21.04.1993
(51) Int. Cl.: A61B 3/10, A61B 3/12

(54) **RETINAL EYE DISEASE DIAGNOSTIC SYSTEM**
ANORDNUNG ZUM DIAGNOSTIZIEREN VON FUNKTIONSSTÖRUNGEN DER RETINA
SYSTEME DIAGNOSTIC POUR AFFECTION RETINIENNE

(43) Date of publication of application: 13.03.1996
(73) Proprietor: Laser Diagnostic Technologies Inc., San Diego, CA 92127 (US)
(72) Inventor: DREHER, Andreas, W., CA 92029 (US); REITER, Klaus, N., 69117 Heidelberg (DE)
(74) Representative: Jehan, Robert
(86) International application number: PCT/US93/03796
(87) International publication number: WO 94/023641

(56) References cited:
- US-A- 4 838 679
- US-A- 5 058 596
- US-A- 5 152 295
- US-A- 5 177 511

## Description

### BACKGROUND OF THE INTENTION

### FIELD OF INVENTION

The invention relates to a method and apparatus for assessing the thickness, topography and nerve fiber orientation of the retinal nerve fiber layer by measuring the polarization effect of the nerve fiber layer of the retina on a impinging light beam while eliminating the obscuring polarizing effects of the anterior segment of the eye.

### DESCRIPTION OF THE PRIOR ART

The retinal nerve fiber layer is the innermost layer of the human retina, defining the front of the retina consisting of the ganglion cell axons which transmit the visual signal generated by the photoreceptors. The ganglion cell axons (nerve fibers) converge to the optic papilla where the optic nerve is formed, which transmits the bundled visual information from the eye to the brain. Glaucoma and other eye diseases damage these nerve fibers, resulting in loss of vision, or blindness. In order to detect glaucoma early and prevent further loss of vision, it is important to assess the condition of the retinal nerve fiber layer as soon and as accurately as possible.

One widely used method of making this assessment employs a fundus camera with red-free illumination to photograph the retinal nerve fiber layer. Blue light (red-free) enhances the visibility of the transparent nerve fibers, and retinal locations with a nerve fiber layer defect appear darker than normal. However, no quantitative results are obtainable with this method.

More recently, several methods have been developed that attempt to quantify the three-dimensional size and shape of the optic papilla, which can be considered a bulk representation of the retinal nerve fibers. By analyzing the topography of the optic papilla and the surrounding retina, an indirect measure of the condition of the retinal nerve fiber layer can be obtained.

One of the current methods is stereoscopic fundus photography wherein two photographs of the fundus are obtained from different angles, and the depth or topography information is extracted by triangulation (see, for example U.S. Patent No. 4,715,703).

Another diagnostic method consists of projecting a stripe or grid pattern onto the fundus, which is observed at a specific angle. An algorithm is used to calculate the topography from the apparent deformation of the projected stripes on the illuminated fundus (see, for example, U.S. Patent No. 4,423,931). More recent methods utilize the technique of confocal scanning laser ophthalmoscopy in which a laser beam is scanned across the eye fundus in two dimensions in order to obtain real-time video images on a TV monitor. By focusing the scanning laser beam on different layers of the retina and confocally detecting the light reflected from the fundus, optical section images of the retina can be obtained. These section images are analyzed to obtain the topography of the fundus.

US-A-5,177,511 discloses apparatus for producing images of an object and, in particular, for observing the rear portions of the eye by an illumination device, the light of which is focused on the object to be imaged and which preferably is provided with at least one laser. A scanning device, which produces a scanning motion of the light from the illumination device on the object to be imaged. A detector device has at least one detector which receives the light reflected from the object to be imaged an assessment and synchronization unit produces the image from the time-sequential output signal from the detector device.

All of these techniques depend on the intensity of light reflected from the retinal surface as the sole probing tool for determining fundus topography. They are based on the assumption that the point of brightest reflection is at the internal limiting membrane, the interface between the vitreous and the retina. The point of maximum reflection is, therefore, assumed to represent the anterior surface of the nerve fiber layer. In reality, the light detected from the fundus is a mixture of light reflected from the internal limiting membrane and light scattered from deeper layers within the retina. Therefore, the maximum of the total intensity distribution of all light detected from the retina does not coincide with the most anterior surface of the retina, and a false presentation is obtained.

A very significant limitation inherent in these conventional methods is the inability to measure the thickness of the retinal nerve fiber layer in addition to the topography. Topographic maps provide an approximate model from which thickness can be derived, but represent indirect and suggestive evidence only. The nerve fiber layer represents on the order of one-tenth of the total thickness of the retina, and changes in its thickness provide the best indicator of progressing disease. A method of directly measuring the actual thickness of the retinal nerve fiber layer would represent a clearly valuable addition to the diagnostic tools available to the medical diagnostician.

It has been known [see for example, Journal of the Optical Society of America A 2, 72-75 (1985)] that the human retina has certain polarization properties. The instant inventors, in a paper delivered in 1991, [Technical Digest on Noninvasive Assessment of the Visual System, 1991 (Optical Society of America, Washington, D.C., 1991), Vol.1, pp. 154-157], showed that the retinal nerve fiber layer was responsible for the polarizing effect of the retina..

The retinal nerve fiber layer consists of parallel axons which are form birefringent and change the state of polarization of light double-passing it. The thicker the nerve fiber layer, the greater the alteration of the state of polarization of incident light and thus of the reflected beam. This phenomenon creates an opportunity to measure the thickness of the nerve fiber layer by gaging the shift in polarization from incident beam to the reflected beam of a polarized light probe.

The use of polarization shifting as the basis for generating data to map retinal nerve fiber layer thickness has not realized its true potential. A major reason for this is the fact that the cornea and the crystalline lens of the eye also have birefringent qualities, in addition to the fundus. The probe must pass through these layers twice. Therefore the total polarization shift is the sum of the shift caused by double-passing both the nerve fiber layer and the anterior segment of the eye. Without compensating for the polarization effects of the anterior segment, the measurement of the retinal polarization effect for thickness mapping would be of limited value as a diagnostic tool.

According to an aspect of the present invention there is provided a method of detecting eye diseases as specific in claim 1.

According to another aspect of the present invention there is provided apparatus for detecting eye diseases as specified in claim 22.

According to another aspect of the present invention there is provided an anterior eye segment polarisation compensator as specified in claim 30.

The preferred embodiment can provide a method and apparatus for measuring polarization properties of the retina while compensating for the polarization effects of the anterior segment of the eye to produce statistically and clinically meaningful results. The polarization state of light returning from the fundus is detected and compared to the initial state before alteration at the fundus. The degree of alteration substantially direct correlates with the thickness of the birefringent nerve fiber layer.

Furthermore, by neutralizing the polarization effects of the anterior segment of the eye and by the use of polarization-sensitive detection means, the light that has been reflected specularly from the internal limiting membrane at the anterior surface of the nerve fiber layer can be distinguished from the light originating from deeper retinal layers. The conventional tomographic methods described in the BACKGROUND are sharpened considerably when polarization-state-altered light, representing light not reflected right at the surface of the fundus but at deeper retinal layers, is filtered out before the maximum amplitude calculations are executed.

In addition to measuring thickness and topography, the system can be used to produce a nerve fiber orientation map. The nerve fibers are arranged in a generally radial pattern, with the optic papilla at the center. Local regions of the nerve fiber array are substantially parallel and exhibit birefringence with the optic axis of the array parallel to the fibers. If the polarization axis of the incident light is rotated, the return beam will show minimum polarization change when the optic axis of the nerve fiber layer, running parallel to the nerve fibers, is parallel to the polarization axis of the incident beam. Because resolution in the order of magnitude of the invention had not been possible in the past, the diagnostic value of this information is of unknown, but it is believed that it will prove useful, especially in conjunction with other tests. A fiber orientation map makes it possible for the first time to trace specific nerve fibers from the optic papilla to their origin. This, for example, allows for the association of blind spots scattered throughout the visual field with specific nerve fibers close to the optic papilla.

To achieve these objectives, a polarized light probe is used in conjunction with a corneal polarization compensator to diagnose the ocular fundus while neutralizing the polarization effects of the anterior segment of the eye. The corneal polarization compensator comprises of a variable retarder through which monochromatic polarized laser light is passed and focused through the cornea onto either the posterior or anterior surface of the lens of the eye. The reflected light double-passes the anterior segment of the eye, traveling back through the variable retarder and is confocally detected. The light is photoelectrically converted, and the signal is processed to control the retardation of the variable retarder in a closed feedback mode. The optical path in the compensation scheme is such that when the variable retarder is adjusted to the point where it neutralizes the polarization distortion of the cornea and lens, the signal at the photodetector is at its maximum and the variable retarder is fixed at this setting.

### BRIEF DESCRIPTION OF THE DRAWINGS

Figure 1 is a diagrammatic section taken through line 1-1 of Figure 1a;
Figure 1a is a diagrammatic view of the eye identifying parts of the anterior segment;
Figure 2 illustrates diagrammatically the main parts of a principle embodiment of the corneal polarization compensator using an ellipsometer;
Figure 3 illustrates diagrammatically one manner in which the nerve fiber layer thickness is mapped with the use of a sequential array of polarizers of different states of polarization;
Figure 4 illustrates a topographical mapping system;
Figure 5 illustrates the appearance of the retinal nerve layer under illumination with linearly polarized light and detection with a crossed polarizer, corneal birefringence being eliminated;
Figure 6 is identical to Figure 5, but illustrating measurement taking place with the orientation of the polarization axis of the illuminating beam and detection filter being rotated about 45 degrees; and,
Figure 7 is a diagrammatic illustration of a photodetector incorporating a focusing lens and a pinhole diaphragm for use in confocal detection techniques.

### DETAILED DESCRIPTION OF THE PREFERRED EMBODIMENT

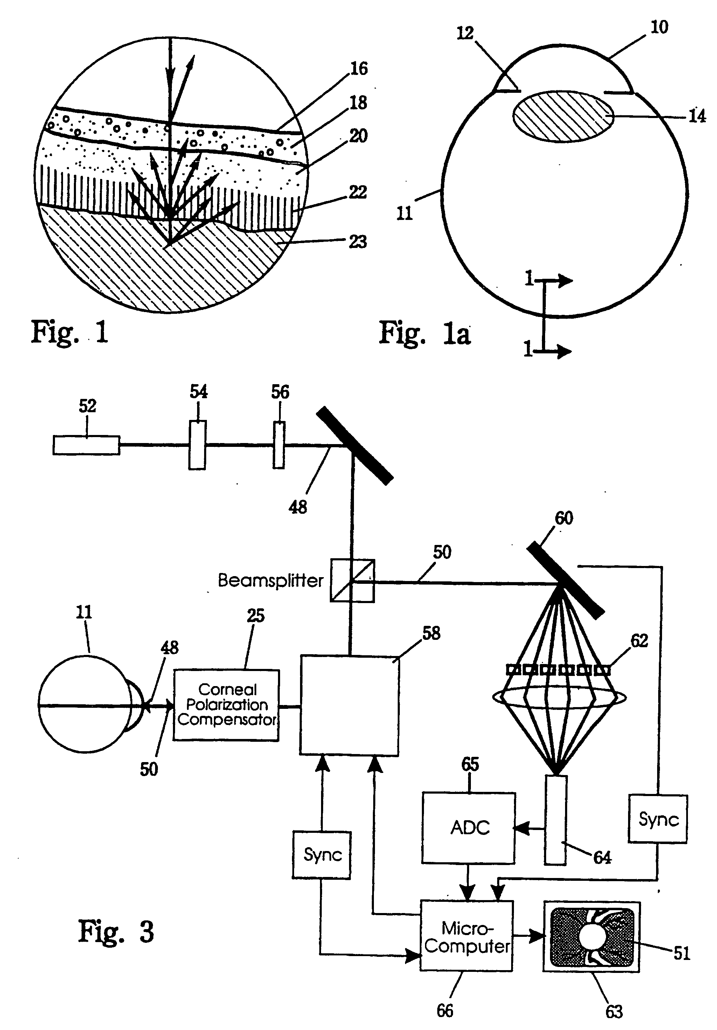

Figures 1a and 1 illustrate the eye 11, in which the cornea 10 serves as the foremost, transparent portion of the eye, behind which is the iris 12 and the lens 14. The interior of the eye 11 is filled with vitreous and at the back of the eye is the retina, composed of the layers illustrated in Figure 1, including the internal limiting membrane 16, the nerve fiber layer 18, the receptor system 20, the retinal pigment epithelium 22, and the choroid 23. All eye structure forward of the membrane 16 is considered the anterior segment of the eye for purposes of this disclosure and claim definitions.

The invention concerns itself primarily with the cornea, the lens, and the nerve fiber layer 18. It is this nerve fiber layer's topographic and thickness measurements which are crucial to the diagnosis of certain diseases, among them being glaucoma. The orientation of the fibers is also useful to a general understanding of a particular eye, and in interpreting the thickness and tomograph data.

As indicated above, the nerve fiber layer 18 has birefringent properties. A polarized light ray incident on the surface of a birefringent medium, with its optic axis parallel to the surface of the medium, will split into two rays of different polarization states, propagating in the same direction but with different velocities. The difference in travelling velocity causes a shift in phase between the two exiting rays. This is called "retardation", and results in altering the polarization of the light. The thicker the birefringent medium, the greater is the retardation of transmitted light. A so-called "quarter wave" retarder incorporates a birefringent medium that retards one of the rays 90 degrees relative to the other, converts linear polarization to circular polarization, and vice-versa.

The nerve fiber layer 18 has the property of birefringence. The cornea and the lens also have birefringent properties, although the birefringence of the lens is small compared to the cornea. There are no other known birefringent layers in the eye.

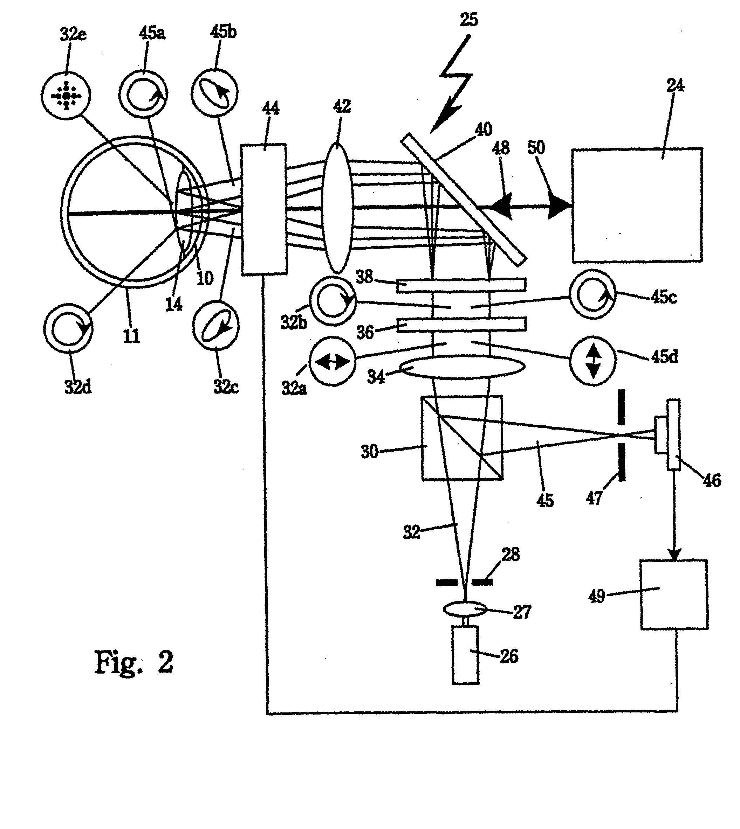

Turning now to Figure 2, a complete system for diagnosing the thickness of the nerve fiber layer is diagrammatically shown. All of the structure in Figure 2 except for the ellipsometer 24 is for the purpose of compensating for the polarization shifting caused by the cornea and lens. (In this disclosure, polarization "shifting" or "alteration" refer to all types of polarization changes, including rotation of the polarization axis of polarized light, the change of linear to elliptical or circularly polarized light or vice-versa, change in the polarization level, and any combination of these). The term "corneal polarization compensator" is used for describing the device for compensating for the polarization effect of the anterior segment of the eye.

The ellipsometer 24 is an instrument which accurately identifies the polarization state of a light beam. In this application, it makes possible the assessment or the nature and degree of polarization state shifting of light which double-passes the nerve fiber layer. This shift correlates to the thickness of the nerve fiber layer once the corneal polarization compensation has been effected. The thinner this layer is, the more advanced is the eye disease, as a general rule.

The corneal polarization compensator 25 utilizes a laser diode 26 which provides a beam of light that is focused by a lens 27 onto the pinhole 28 and expands as a cone until it impinges upon the polarizing beamsplitter 30. This beamsplitter has two purposes, the first of which is to polarize the incident compensation beam 32, which it does as is indicated by the legend indicated at 32a, illustrating the linear transverse polarization that the beam has at this point. The beam subsequently passes through a collimating lens 34 and a quarter wave retarder 36, which converts the beam 32 from linear polarization illustrated in the legend 32a to the clockwise circular polarization indicated in the legend 32b.

At this point, the incident compensation beam 32 passes through a reticulated or rectangular diffraction grating 38, which has the effect of splitting the light into a plurality of beams, so that a plurality of focus points as indicated at 32(e) are used by the compensator rather than a single spot. The beam is reflected on the beamsplitter 40, converged by the converging lens 42, and passed through the variable retarder 44, which in the preferred embodiment is a liquid crystal retarder. This retarder changes the polarization of the incident beams from circular polarization to elliptical as illustrated at 32c, still being clockwise in sense.

At this point, the plurality of converging sub-beams of the whole beam 32 from the variable retarder 44 converge, passing through the cornea 10 and lens 14, becoming circularly polarized as indicated at 32d and reflecting as return compensation beam 45 from the posterior surface of the eye lens 14, as illustrated. This reflected or return compensation beam is polarization - shifted by the double-passage through the cornea and lens not only to circular polarization as indicated at 32d, but is shifted to reverse the direction of the circular polarization as a result of the reflection, as indicated at 45a. (For purposes of the claims, the incident and return beams are each treated singularly, but each includes all of the composite beams split out by the diffraction grating and then re-converged).

The return compensation beam 45 has the polarization states illustrated in the legends 45a-45d, above and to the right of the configuration. Immediately upon reflecting from the lens surface, the right-hand circular polarization is changed to left-hand circular polarization 45a, and shifts to elliptical polarization as indicated at 45b upon passage through the cornea 10 and lens 14 The return compensation beam 45 passes through the variable retarder 44 where its polarization is restored to circular polarization as indicated in 45c, and travels back through the elements that the impinging beam went through, passing through a polarization shift at 45d until the beam arrives at the polarizing beamsplitter 30.

It will be remembered that when the beam initially passed up through this beamsplitter, it was transversely polarized as indicated at 32a. It is a property of a polarizing beamsplitter to transmit light that is polarized perpendicularly to its reflecting surface, and to reflect light that is polarized parallel to its reflecting surface. As the return compensation beam is now completely linearly polarized, parallel to the reflecting surface of the beamsplitter 30, the return compensation beam 45 is reflected to the right, towards the photodetector 46. The return compensation beam is focused by the lens 34 onto the pinhole 47 in front of the photodetector 46. The pinholes 47 and 28 are located in optically conjugate planes to the focal points formed at the posterior surface of the lens. This confocal arrangement assures that stray light reflected from other areas than the focal points is blocked by the pinhole 47 and cannot reach the photodetector 46.

In other words, when all light of the return beam 45 impinging downward upon the polarizing beamsplitter 30 is linearly polarized orthogonally to the direction of the upwardly travelling beam 32, all of the light reflected from the surface of the lens 14 would travel through to the photodetector 46. Thus, with no polarization shift at all caused by the anterior segment of the eye, incident and return compensation beams 32 and 45 would have the polarization states shown at 32a and 45d, respectively. The variable retarder is adjusted to maximize the intensity of light in the polarized state shown at 45d as closely as possible.

The photodetector 46 outputs a voltage signal corresponding to light intensity that feeds back into the circuit 49. Because the cornea and lens shift the polarization, the variable retarder is varied by the circuit 49 until the electric signal coming from the photodetector 46 is maximized. Figure 2 illustrates states of polarization of incident and return beams after the compensator has already been adjusted to compensate for anterior segment polarization shift. After the variable retarder 44 has been adjusted for the optimal compensation of corneal and lenticular polarization distortion, the ellipsometer 24 is free to pass its incident diagnostic beam 48 through the beamsplitter, having its beam polarization-compensated by the variable retarder (compensator) 44, and receive a return beam 50 that actually reflects not the polarization distortion caused by the cornea and lens, but only that of the nerve fiber layer in question. This polarization information is then captured and can be analyzed according to ellipsometry techniques that are known in the prior art or as set forth in this disclosure.

This process has been disclosed having the incident and return compensation and diagnostic beams double-passing the variable retarder 44. However, only one of the compensation beams and one of the diagnostic beams would have to pass through the variable retarder, either the incident or return beam. The simplest geometry producing the most accurate results involves double-passing both beams as shown.

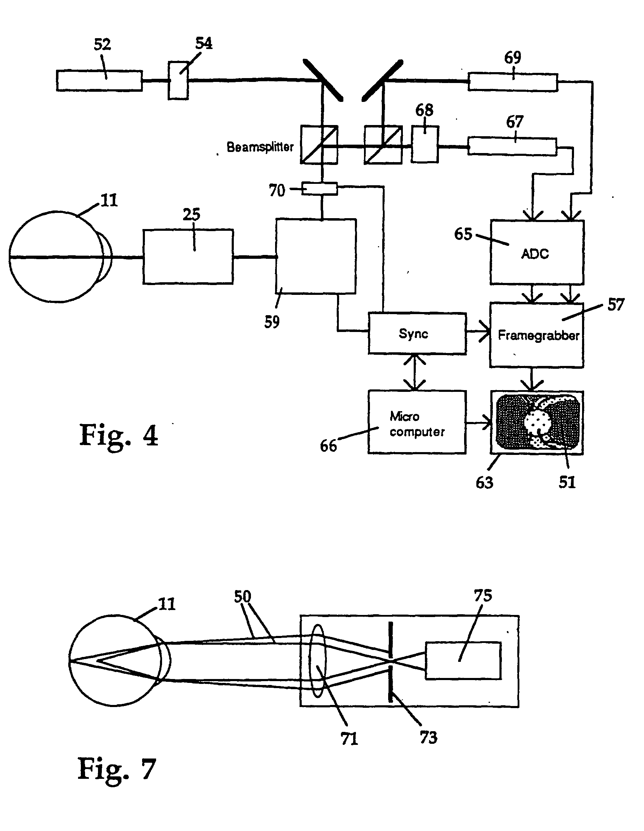

The corneal polarization compensator 25 is used in all of the techniques that are discussed in this disclosure. It has already been stated that the ellipsometer can be used basically by itself, as shown in Figure 2, along with scanning and analysis equipment, not shown in Figure 2, to provide a useable map of the thickness of the retinal nerve fiber layer. A computer frame 51 shown in Figures 3 & 4 illustrates the appearance of a typical nerve fiber layer thickness or topographic map.

One way of measuring and mapping the thickness of the nerve fiber layer is shown in Fig. 3, with a system that uses a custom ellipsometer made for this use. It produces an incident diagnostic beam 48 generated by the laser 52, subsequently linearly polarized by linear polarizer 54, converted to circular polarization by quarter-wave retarder 56 and scanned across the ocular fundus by the scanning unit 58. At each point of the scan, the return diagnostic beam 50 is then again scanned by an oscillating mirror 60 sequentially across a plurality of polarizers 62 forming an array. Six polarizers are shown in the array of Figure 3, and as the return beam reaches the detector 64 in sequence from each of the polarizers the beam intensity is photoelectrically converted by the detector 64 into a signal that is digitized by an ADC (Analog-to-Digital converter) 65 and stored in the memory of the computer 66. From the data stored in the computer, the four elements of the Stokes vector of the incident diagnostic beam 48 are compared to the calculated Stokes vector of the return diagnostic beam, and the change in polarization at the current measuring location is displayed on the CRT display 63. Subsequently, the incident diagnostic beam is guided by the scanning unit 58 to the next measuring site.

The scanned polarizer system of Figure 3 is diagrammatic, and the polarizers could be either reflective or transparent and would ordinarily have a mirror system converging the respectively produced beams onto the detector. For every point scanned on the ocular fundus, all of the polarizers 62 would be scanned by the oscillating mirror 60.

It would be clear to a person skilled in the art that the principle described can also be performed by changing the time sequence of the polarization data measurement process. For example, instead of scanning a single point at 58 while mirror 60 undergoes a complete scanning cycle, the incident diagnostic beam 48 could first be scanned by the scanning unit 58 over the whole examination area, while the return diagnostic beam 50 is fixed on one of the polarizers, then on to the next. Either way, the data points are aggregated and displayed as an intensity- or color-coded map, for example. Also, illumination of the examination area with a scanning laser could be modified by illuminating the fundus with a static (non-scanning) light source and replacing the detector 64 with a camera.

Thus far, gaging of the thickness of the nerve fiber layer, and the creation of a thickness map display has been discussed. Using a similar technique, a topographic map can be made which is substantially more accurate and detailed than those made with conventional techniques.

Figure 4 illustrates a system similar to the Figure 3 setup, which will produce a topographic map of the anterior surface of the retinal nerve fiber layer. The scanning unit 58 is replaced by a three-dimensional scanning unit 59, and the detector 64 is replaced by a confocal detection unit 67. It is similar to the typical confocal system that is now used, except that the optical data that is received back from the nerve fiber layer is sorted by discarding (filtering out) any data, (any light rays) that are returning from the eye having altered polarization. Because the corneal polarization compensator neutralizes polarization shifting caused by the anterior segment of the eye, and the polarization state of the incident light beam is known, any return light which does not match the incident beam in its state of polarization is known to have been reflected from a surface deeper than the nerve fiber layer surface 16. Conventional confocal topographical mapping is enhanced by discarding this light information, which represents false data. Mechanically this is done by scanning across the entire surface of the nerve fiber layer in progressively deeper focal planes. and generating an intensity map, and repeating for consecutively deeper layers. The analyzer 68 includes a filter polarized parallel to the incident beam, attenuating light of other polarization states, and the computer stores an intensity map for each plane. These maps are software-overlaid, and the brightest return plane for each point across the fundus is considered to be the depth of the front of the nerve layer at that point. This can actually be done with a single scan by using two confocal detectors focused just to the far and near sides of the anterior surface, respectively, ad interpolating from the relative intensities at each point.

The potential information that can be gleaned from the interior of the eye utilizing corneal compensation is considerable. For example, topographic maps of deeper layers of the eye than the surface of the nerve fiber layer can be made by rejecting the light in the polarization state of the initial beam, rather than vice-versa.

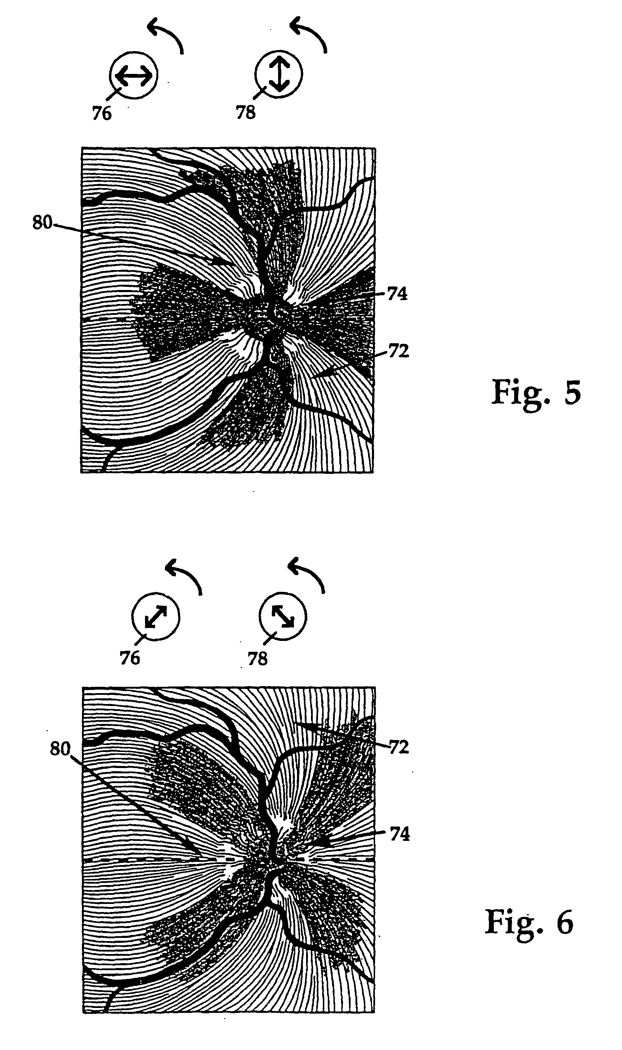

Returning from tomography to thickness mapping again, the same setup shown in Figure 4 used for topographic map-making can be used to produce an enhanced nerve fiber thickness map. A polarization rotator 70 is interposed in the light path of the incident or return diagnostic beam, or both. A second detector 69 measures the absolute intensity of the return diagnostic beam independent from its polarization state. Referring to Figures 5 & 6, the retinal nerve fiber layer 14 comprises an array of radially arranged nerve fibers 72 which converge to form the optic papilla 74. The fibers are about half the diameter of the wavelength of visible light in width. Because the array exhibits local parallelism and wavelength-order-of-magnitude spacing, it exhibits directional birefringence.

It is illuminated with linearly polarized light, and the reflected light from the fundus is passed through an analyzer with an orthogonally polarized filter 68 to a photodetector or collector. The states of polarization of the incident beam and the filter are diagrammed at 76 and 78. A "cross" pattern of brightness, indicated at 80, will appear at the detector. There will be darkness along the polarization axes of both the incident light beam and the analyzer filter. The bright arms correspond to areas of the nerve fiber layer having fiber orientation rotated 45 degrees to either side of the polarization axis of the incident beam and the analyzer filter. The bright portions of the cross provide an accurate indication of the thickness of the nerve fiber layer at these points, as substantial change in polarization caused by substantial nerve fiber layer thickness will shift the polarization of the light adequately to pass through the analyzer polarization filter.

In order to obtain a best measurements, the polarization axes of the incident beam and analyzer filter are synchronously rotated through 90 degrees, which constitutes a complete rotation cycle, with a brightness reading taken about every 2 degrees, for every point on the fundus that will appear on the map. The polarization axis can be held at one orientation (actually rotating through 2 degrees) while the entire fundus is scanned and then "incremented" 2 degrees for the next scan until all test orientations of the polarization axis have been sampled for the entire field. or, in reverse, completing a full polarization axis rotation cycle at each point on the fundus before moving on.

The brightest return beam is thus picked up for every point in the field. These brightest points are cumulated and formed into an intensity map corresponding point-to-point to the relative thickness of the fundus.

The second photodetector 69 is used to measure the total amount of reflected intensity of the return diagnostic beam at the corresponding points on the fundus. By normalizing the intensity values obtained with the first photodetector 67 with the corresponding intensity values obtained with detector 69, absolute changes in the state of polarization of the return diagnostic beam are calculated. This permits variations in return beam intensity caused by factors other than polarization shifting to be factored out of the final data.

A substantially identical technique with different computer handling of data produces a nerve fiber orientation map. The orientation of maximum return beam intensity at each point represents alignment of the beam and filter polarization axes with the optic axis of the nerve fiber layer.

In summary, using the illustrated systems and described methods, three basic types of measurements are possible, producing three different maps. These are, (1) nerve fiber layer surface topography, (2) nerve fiber layer thickness, and (3) nerve fiber orientation.

The first measurement produces improved results over existing techniques, whereas the second and third techniques, thickness and fiber orientation mapping, represent new tools in eye disease diagnosis and, in many cases, provide clinically significant and useful data for the first time.

Two detector systems are shown, the ellipsometer of Figure 2 and the 6-polarizer array of Figure 3 (actually just another way to make an ellipsometer). Either could be used in any of the described techniques, and many other configurations can be arranged.

Any of the setups can be modified for confocal detection or not, confocal detection only being necessary in tomographic mapping. Modulation of one or both of the incident and return modulation beams, by rotation of the polarization axis produces more accurate and highly resolved thickness maps, and is necessary in fiber orientation mapping, but is less useful in tomography as light altered at all in its polarization state is discarded.

The feasibility of all of the disclosed diagnostic techniques and equipment depends on the polarization characteristics of the ocular fundus, and further depend on the compensating capability of the corneal polarization compensator to produce the most useable results. These polarization-based diagnostic techniques contribute substantially to repertory of tools and techniques used to accurately diagnose diseases of the eye, and especially for the early diagnosis of glaucoma.

The first technique results in topographic images which are greatly enhanced in resolution and accuracy compared to topographic maps produced by currently used methods. The second and third procedures, nerve fiber layer thickness mapping and fiber orientation map production, go beyond improvements to existing techniques and represent new tools in eye disease diagnosis. The results of these tests provide information previously unavailable to the medical profession. For the first time, detailed, high-resolution, accurate displays of the nerve fiber layer thickness, the wellspring of glaucoma diagnosis source data, and a map tracing the actual physical connection between specific nerves and blind spots in the field of vision characteristic of optic nerve deterioration, are available to the diagnostician.

### DEFINITIONS OF TERMS USED IN THE DESCRIPTION AND CLAIMS

The following definitions and statements set forth the meaning of the defined words and phrases as used in this specification and in the appended claims:
**ABSOLUTE INTENSITY** refers to the sum of the intensities of all of the component parts of a light beam, including polarized and unpolarized segments.
**ANALYZER, or POLARIZATION ANALYZER:** a device whose output is a function of the polarization state of analyzed light in some way. It may be a bare polarization filter. An ANALYZER may or may not produce results which are directly readable by the operator.
**ANTERIOR SEGMENT OF THE EYE** refers to all parts of the eye forward of the OCULAR FUNDUS, in this instance those parts which pass light incoming through the cornea. It includes the vitreous, lens, aqueous, and cornea and any membranes.
**BIREFRINGENCE** is a POLARIZATION PROPERTY of certain materials which retards the propagation velocity of only part of a transmitted beam, causing it to have a phase lag with the rest of the beam, shifting the polarization phase; birefringence is not the only possible polarization property.
**FUNDUS** = ocular fundus.
**KNOWN STATE OF POLARIZATION** refers to a POLARIZATION STATE that is controlled such that interaction with equipment such as polarization filters produces meaningful and sometimes measurable results. The phrase does not mean that the operators necessarily know what the polarization state is at a given time.
**MODULATION of the polarization state** of light is the alteration of the polarization state over time analogous to frequency or amplitude modulation; the retardation can be modulated, which if executed through a complete 360° cycle causes the polarization to cycle through linear, elliptical, circular, elliptical, linear, reverse-direction elliptical, circular, elliptical and back to linear. Or, the polarization axis can be modulated by being rotated about the optical axis. Any alterable polarized condition in which the alteration is detectable could be modulated.
**OCULAR FUNDUS:** generically the layers of the eye posterior to the internal limiting membrane covering the anterior surface of the nerve fiber layer (primarily the retina and the sclera); the anterior surface of the internal limiting membrane separates the ANTERIOR SEGMENT and the FUNDUS, as the terms are used in this disclosure.
**POLARIZATION AXIS.** Light behaves as a transverse wave in which the waves vibrate perpendicularly to the direction of propagation. The polarization axis of light is oriented in the direction of vibration, orthogonal to the propagation direction.
**POLARIZATION EFFECTS** on light refers to alterations made to the polarization state of incident light by objects and media as a result of their POLARIZATION PROPERTIES;
**POLARIZATION PROPERTIES** refers to the characteristics of specific materials and structure relating to the POLARIZATION EFFECTS they have, or do not have, on the POLARIZATION STATE of incident light, such as polarizing unpolarized light, rotating the POLARIZATION AXIS, affecting the degree or type of polarization, or not affecting polarization at all.
**REVERSAL OF SENSE OR DIRECTION** of polarized light: light reverses, left-hand/right-hand, the polarization sense when it is reflected from a specular surface.

## Claims

1. A method of examining the polarization properties of the fundus of the eye while substantially compensating for the polarization effects of the anterior segment of the eye, comprising the following steps:
(a) illuminating the fundus including the retinal nerve fiber layer through the pupil and anterior segment including the cornea and lens, with at least partially polarized incident light in an incident diagnostic beam (48) of a known polarization state;
(b) detecting reflected light reflected from the fundus from the incident diagnostic beam as a return diagnostic beam (50) with a polarization-sensitive detection device ; and
(c) substantially compensating for the polarization effects of the anterior segment of the eye through which said diagnostic beams (48,50) pass by using an anterior eye segment polarization compensator comprising a variable retarder through which a compensation beam of polarized light is passed and focussed through the cornea onto a surface of the lens of the eye, the compensation beam reflected at said lens surface being again passed through said variable retarder, and compensator monitoring means operable to determine the degree of combined polarization change of the compensation beam due to its double passage through the retarder and the anterior segment of the eye, such that the state of polarization of said return diagnostic beam (50) can be analyzed and compared to said known polarization state of the incident diagnostic beam to determine the degree of alteration of the state of polarization of said return diagnostic beam (50) based on the net polarization effect of the fundus, and determining said net polarization effect of the fundus from said degree of alteration, from which an inference can be made regarding the presence and progress of certain eye diseases.

2. A method according to claim 1, wherein step (c) is accomplished by the use of an anterior eye segment polarization compensator interposed in the propagation path of at least one of said beams and which provides said variable retarder (44).

3. A method according to claim 2, wherein step (c) comprises adjusting said variable retarder (44) to achieve a known amount of combined net retardation induced in light passing through said variable retarder and the anterior segment of the eye.

4. A method according to claim 3, wherein step (c) is executed by adjusting said variable retarder (44) such that the combined net retardation of said variable retarder and the anterior segment of the eye is substantially null such that retardation of the return diagnostic beam detected in step (b) indicates substantially as closely as possible retardation caused by the fundus independent from any retardation of the anterior segment of the eye.

5. A method according to claim 4, wherein step (C) is accomplished by focusing an incident compensation beam (32) on a surface of the lens of the eye producing a reflected compensation beam, passing both of said compensation beams through said variable retarder (44) and adjusting said variable retarder (44) until the compensation beams on the side of the variable retarder remote from the eye are both in substantially identical states of polarization, except for a 180° phase shift caused by the reflection at the surface of the lens of the eye.

6. A method according to claim 5, wherein said incident compensation beam is laser-produced and linearly polarized with a first polarization axis and passed through a quarter-wave retarder (36), said reflected compensation beam returns through said quarter-wave retarder (36) to become linearly polarized with a second polarization axis orthogonal to said first polarization axis absent the polarization effects of the anterior segment of the eye and variable retarder, and said variable retarder (44) is adjusted so that the state of polarization of the reflected compensation beam after passing through the quarter-wave retarder (36) as closely as possible resembles a state of linear polarization with a polarization axis orthogonal to said first polarization axis.

7. A method according to any preceding claim, wherein said polarization sensitive detection device of step (b) is an ellipsometer (24) and step (b) is executed by operating same.

8. A method according to claim 7, and including the steps of scanning said incident diagnostic beam across said fundus to a plurality of geometric points of incidence and mapping the degree of alteration of the state of polarization of said return diagnostic beam as a function of said geometric points of incidence.

9. A method according to claim 8, wherein at least one of said diagnostic beams is modulated in its state of polarization to produce a sequence of modulated polarization states, and for each such modulated polarization state of said at least one of said diagnostic beams said return diagnostic beam is analyzed for each of said illuminated points of the fundus to derive a polarization indicator for each of said illuminated points, and the polarization indicators for all illuminated points of the fundus for each of said modulated polarization states are stored in a computer memory (66) as modulated-polarization-state-specific images, and including the step of calculating the degree of alteration of the state of polarization of said return diagnostic beam from the modulated polarization-state-specific polarization images stored in the computer memory for each illuminated point of the fundus, and including the step of mapping said degrees of alteration of the state of polarization of said return diagnostic beam as a function of the illuminated points of said fundus.

10. A method according to any preceding claim, wherein said polarization sensitive detection device comprises a detecting scanner (58) which scans said return diagnostic beam sequentially through a plurality of polarizers (62) of different polarization states and including the steps of sequentially intensity-analyzing said return diagnostic beam after it is sequentially scanned through each of said plurality of polarizers (62) to produce a polarizer-specific intensity value for each of said polarizers from which the state of polarization of said return diagnostic beam can be calculated.

11. A method according to claim 10, wherein said incident diagnostic beam illuminates said fundus to generate a plurality of illuminated points, and said return diagnostic beam from each illuminated point of said fundus is collected through one polarizer of said plurality of polarizers (62) resulting in a polarizer-specific polarization image, and said polarizer-specific polarization image is stored in a computer memory (66), and including the step of repeating the immediately above-stated procedures of illuminating said fundus, collecting said return diagnostic beam, and storing said polarizer-specific polarization image, for each polarizer of said plurality of polarizers (62), and including the step of mapping the degree of alteration of the state of polarization of said return diagnostic beam for each image point of said polarization images stored in said computer memory.

12. A method according to claim 1, and including a diagnostic scanner (58) scanning the incident diagnostic beam sequentially across said fundus to a plurality of geometric points of incidence on said ocular fundus and including the step of mapping the degree of alteration of the state of polarization of said return diagnostic beam as a function of said geometric points of incidence scanned on said fundus.

13. A method according to claim 1, wherein said retinal nerve fiber layer exhibits birefringence and has an anterior surface and a posterior surface, and step (a) includes substantially focusing said incident light on a retinal layer generally posterior to said posterior surface of the nerve fiber layer, and step (b) comprises detecting the degree of alteration of the state of polarization of said return diagnostic beam caused by the birefringence of the retinal nerve fiber layer, and including the step of transforming said degree of alteration of the state of polarization of said return diagnostic beam into a nerve fiber layer thickness reading and the step of displaying said nerve fiber layer thickness reading as an image correlating brightness with the thickness of said retinal nerve fiber layer to define a mapped nerve fiber layer thickness image.

14. A method according to claim 1, wherein said retinal nerve fiber layer has an anterior surface, the method including the step of focally scanning said incident diagnostic beam across said anterior surface in at least one focal plane with a diagnostic scanner, and the step of creating a topographic map of said anterior surface by mapping confocally detected scanned points reflected from the fundus displaying minimal retardation in the reflected light.

15. A method according to claim 14, wherein said creating step includes filtering out polarization-shifted light by passing said return diagnostic beam through a polarized filter (30) having an effective polarization axis parallel to the polarization axis of said incident diagnostic beam.

16. A method according to claim 1, wherein said incident diagnostic beam has a first polarization axis, and said return diagnostic beam has a second polarization axis, and step (a) includes rotating the polarization axis of at least one of said diagnostic beams to create a plurality of different sequential polarization state relationships among said diagnostic beams and the birefringent retinal nerve fiber layer.

17. A method according to claim 16, including the steps of analyzing said reflected light and producing an intensity map of the retinal nerve fiber layer which correlates the maximum level of retardation achieved throughout the rotation of the polarization axis of at least one of said diagnostic beams at each illuminated point on the retinal nerve fiber layer with a corresponding light intensity level on said map.

18. A method according to claim 17, wherein said fundus contains an optic papilla with a center, the method including the steps of analyzing said reflected light at fundus locations along at least one line which passes through said center of said optic papilla and is oriented at a fixed angle with respect to the rotating polarization axis of one of said diagnostic beams.

19. A method according to claim 16, 17, or 18, including the step of scanning said incident diagnostic beam across said fundus.

20. A method according to claim 1, wherein said retinal nerve fiber layer consists of nerve fibers which demonstrate substantial local parallelism at local regions thereacross, and said retinal nerve fiber layer exhibits birefringence with an optic axis substantially parallel to the orientation of said nerve fibers, step (a) including illuminating said fundus to illuminate at least some of said local regions of said nerve fiber layer, and step (b) including determining the optic axis of said nerve fiber layer in each of said local regions and the step of deriving the orientation of the nerve fibers from said optic axis in said at least some of said local regions.

21. A method according to claim 20, wherein said incident diagnostic beam has a first polarization axis, and said return diagnostic beam has a second polarization axis, and step (a) includes rotating the polarization axis of at least one of said diagnostic beams through substantially 90° to generate a plurality of polarization axis orientation states, and step (c) includes determining the degree of alteration of the state of polarization of said return diagnostic beam as a function of the rotational angle of said polarization axis orientation states at each of said local regions of said nerve fiber layer at each of said local regions by identifying the rotational angle of the polarization axis orientation state which produced the minimum degree of alteration of the state of polarization of said return diagnostic beam.

22. Apparatus for detecting eye diseases comprising:
(a) a polarized light source (26) for producing an incident diagnostic beam (48) of known state of polarization;
(b) transmitting means (42,44) for transmitting said incident diagnostic beam into an eye through the pupil, where it is reflected from the interior of the eye as a return diagnostic beam (50);
(c) optical collecting means (40) for collecting said return diagnostic beam and transmitting same to a polarization sensitive detection device (24);
(d) a polarization sensitive detecting device (24) for collecting and transducing information about the state of polarization of said return diagnostic beam into an electrical signal such that the intensity of said electrical signal is a function of the state of polarization of the return diagnostic beam;
(e) a corneal polarization compensator comprising a variable retarder through which a compensation beam of polarized light is passed and focussed through the cornea onto a surface of the lens of the eye, the compensation beam reflected at said lens surface being again passed through said variable retarder, and compensator monitoring means operable to determine the degree of combined polarization change of the compensation beam due to its double passage through the retarder and the anterior segment of the eye, thereby to modify the polarization of at least one of said diagnostic beams such that the combined polarization effects on said diagnostic beams of both the anterior segment of the eye and the corneal polarization compensator are known, permitting accurate assessment of any alteration of the polarization state of said return diagnostic beam caused by the polarization properties of the posterior portion of the eye.

23. Apparatus according to claim 21, including means (34-42) operable to modulate the polarization of at least one of said diagnostic beams.

24. Apparatus according to claim 21 or 22, wherein said transmitting means (42,44) is operable to focus said incident diagnostic beam (48) on one focal point in the eye and including a point orifice diaphragm positioned conjugate to said focal point in front of said polarization sensitive detecting device to restrict said detector to detecting only light reflected from objects at said focal point from said incident diagnostic beam.

25. Apparatus according to claim 21 or 23, wherein said transmitting means includes a diagnostic scanner (58) operable to scan said incident diagnostic beam across the ocular fundus of the eye.

26. Apparatus according to claim 24, wherein said transmitting means includes focusing means operable to change the focal point of said incident diagnostic beam.

27. Apparatus according to claim 21, wherein said polarization sensitive detecting device comprises an ellipsometer (24).

28. Apparatus according to claim 21, 24 or 25, wherein said polarization sensitive detective device comprises at least two detectors (24,46,64) operable to detect substantially at least two different polarization components of said return beam.

29. Apparatus according to claims 21, 24 or 25, wherein said polarization sensitive device comprises an array of polarizers (62) of different pre-determined states of polarization, a deflecting device (60) for scanning said return diagnostic beam sequentially onto said polarizers (62), collector means (64) for sequentially receiving light from said polarizers, at least one detector being operable to receive light from said collector means and transducing the intensity of said return diagnostic beam into electrical signals of intensity corresponding to the polarization of the light from said polarizers.

30. An anterior eye segment polarization compensator for use in a diagnostic system using polarized light in the diagnosis of eye disease, comprising:
(a) a source of light (26) for producing an incident compensation beam (48) in a known state of polarization;
(b) transmitting means (40,42) for directing said incident compensation (48) beam through the anterior segment of the eye to be reflected by regions of the eye more anterior than the cornea, to form a reflected compensation beam (50) exiting the eye along an optical pathway substantially aligned with the incident compensation beam;
(C) a variable retarder (44) optically interposed in said optical pathway; and
(d) compensator monitoring means operable to determine the degree of combined polarization shift from said incident compensation beam as it double-passes the variable retarder (44) and the anterior segment of the eye to exit said variable retarder (44) as said reflected compensation beam (50), such that any alteration of the state of polarization of said reflected beam from the state of polarization of said incident beam caused by the anterior segment of the eye can be neutralized by adjusting said variable retarder.

31. A compensator according to claim 30, wherein said incident compensation beam (50) is linearly polarized by being passed through a polarizing beamsplitter (30) from a first side thereof, and said reflected beam is reflected from a second side of said polarizing beamsplitter (30) and focused on a photodetector (46) and including a quarter-wave retarder (36) in said optical passageway such that when said variable retarder (44) is set properly, said photodetector (46) detects maximum intensity when said variable retarder (44) has compensated for the polarization of said anterior segment of the eye.

32. A compensator according to claim 30 or 31, including a reticulated diffraction grating (38) disposed in the incident compensation beam to fragment said incident compensation beam into a plurality of sub-beams.

33. A compensator according to claim 30, 31 or 32, including a polarization sensitive device comprising an ellipsometer (24) operable to emit an incident diagnostic beam (48) along a path through said variable retarder (44) and which is reflected as a return diagnostic beam (50), said diagnostic beams and said compensation beams having substantially coincident paths through the variable retarder (44) and the anterior segment of the eye, such that the return diagnostic beam is automatically polarization-state adjusted to compensate for any alteration of polarization state caused by the anterior portion of the eye.

34. A compensator according to claim 33, wherein said polarization sensitive device (64) includes a light source for producing a polarized beam, and means to scan said polarized beam across the retinal nerve fiber layer of the eye, causing the reflection of a return diagnostic beam, and including an array of polarizers (62) of progressive levels of polarization which are sequentially illuminated by said return diagnostic beam (50), and means for directing said return diagnostic beam, after passing said polarizers, to said polarization sensitive detection device (64) for detection of the state of polarization.

35. A compensator according to claim 30, including a polarization sensitive device comprising a tomographic probe provided with a three-dimensional scanner (59) and an analyzer and detector to filter out return light reflected from the nerve fiber layer which has been altered in polarization and an analyzer to analyze the return light that has not been altered in polarization, and means (63) operable to display a topographic map of the nerve fiber layer based on an analysis of geometric points of the nerve fiber layer producing non-polarization-altered return light.

## Patentansprüche

1. Verfahren zum Untersuchen der Polarisationseigenschaften des Augenhintergrunds, während Polarisationseffekte des anterioren Augensegments im Wesentlichen kompensiert werden, mit den folgenden Schritten:
(a) Beleuchten des Hintergrunds einschließlich der Nervenfaserschicht der Retina durch die Pupille und das anteriore Segment mit der Hornhaut und der Linse hindurch mit zumindest teilweise polarisiertem einfallendem Licht in einem einfallenden Diagnosestrahl (48) von bekanntem Polarisationszustand;
(b) Erfassen von am Hintergrund reflektiertem Reflexionslicht aus dem einfallenden Diagnosestrahl als zurückkehrendem Diagnosestrahl (50) mittels einer polarisationsempfindlichen Erfassungsvorrichtung; und
(c) im Wesentlichen Kompensieren der Polarisationseffekte des anterioren Augensegments, durch das die Diagnosestrahlstrahlen (48, 50) laufen, unter Verwendung eines Polarisationskompensators für das anteriore Augensegment, mit einem variablen Verzögerer, durch den ein Kompensationsstrahl von polarisiertem Licht läuft, das durch die Hornhaut auf eine Fläche der Augenlinse fokussiert wird, wobei der an dieser Linsenfläche reflektierte Kompensationsstrahl den variablen Verzögerer erneut durchläuft, und einer Kompensator-Überwachungseinrichtung, die so betreibbar ist, dass sie den Grad der kombinierten Polarisationsänderung des Kompensationsstrahls auf Grund seines doppelten Durchlaufs durch den Verzögerer und das anteriore Augensegment bestimmt, so dass der Polarisationszustand des zurückkehrenden Diagnosestrahls (50) analysiert und mit dem bekannten Polarisationszustand des einfallenden Diagnosestrahls verglichen werden kann, um das Ausmaß der Änderung des Polarisationszustands des zurückkehrenden Diagnosestrahls (50) auf Grundlage des Netto-Polarisationseffekts des Hintergrunds zu bestimmen, und um den Netto-Polarisationseffekt des Hintergrunds aus dem genannten Änderungsgrad zu bestimmen, woraus eine Schlussfolgerung hinsichtlich des Vorliegens und des Fortschreitens bestimmter Augenerkrankungen getroffen werden kann.

2. Verfahren nach Anspruch 1, bei dem der Schritt (c) unter Verwendung eines Polarisationskompensators für das anteriore Augensegment ausgeführt wird, der in den Ausbreitungspfad mindestens einer der Strahlen eingesetzt ist und der den variablen Verzögerer (44) bildet.

3. Verfahren nach Anspruch 2, bei dem es zum Schritt (c) gehört, den variablen Verzögerer (44) so einzustellen, dass ein bekanntes Ausmaß kombinierter Nettonacheilung erzielt wird, wie sie in Licht hervorgerufen wird, das den variablen Verzögerer und das anteriore Augensegment durchläuft.

4. Verfahren nach Anspruch 3, bei dem der Schritt (c) dadurch ausgeführt wird, dass der variable Verzögerer (44) so eingestellt wird, dass die kombinierte Nettonacheilung desselben und des anterioren Augensegments im Wesentlichen null ist, so dass die im Schritt (b) erfasste Nacheilung des zurückkehrenden Diagnosestrahls im Wesentlichen so gut wie möglich die Nacheilung anzeigt, die unabhängig von irgendeiner Nacheilung durch das anteriore Augensegment durch den Hintergrund hervorgerufen ist.

5. Verfahren nach Anspruch 4, bei dem der Schritt (c) dadurch ausgeführt wird, dass ein einfallender Kompensationsstrahl (32) auf eine Fläche der Augenlinse fokussiert wird, wodurch ein reflektierter Kompensationsstrahl erzeugt wird, diese beiden Kompensationsstrahlen durch den variablen Verzögerer (44) geschickt werden und dieser eingestellt wird, bis die Kompensationsstrahlen auf der Seite des variablen Verzögerers, die vom Auge entfernt ist, beide im Wesentlichen denselben Polarisationszustand aufweisen, mit Ausnahme einer Phasenverschiebung von 180°, die durch die Reflexion an der Oberfläche der Augenlinse hervorgerufen wird.

6. Verfahren nach Anspruch 5, bei dem der einfallende Kompensationsstrahl durch einen Laser erzeugt wird und er mit einer ersten Polarisationsachse linear polarisiert ist, und er durch einen Viertelwellenverzögerer (36) geschickt wird, der reflektierte Kompensationsstrahl durch den Viertelwellenverzögerer (36) zurückkehrt, um mit einer zweiten Polarisationsachse rechtwinklig zur ersten Polarisationsachse linear polarisiert zu werden, ohne die Polarisationseffekte des anterioren Augensegments und des variablen Verzögerers, und der variable Verzögerer (44) so eingestellt wird, dass der Polarisationszustand des reflektierten Kompensationsstrahls nach dem Durchlaufen des Viertelwellenverzögerers (36) so gut wie möglich dem Zustand linearer Polarisation mit einer Polarisationsachse rechtwinklig zur ersten Polarisationsachse ähnelt.

7. Verfahren nach einem der vorstehenden Ansprüche, bei dem die polarisationsempfindliche Erfassungsvorrichtung im Schritt (b) ein Ellipsometer (24) ist und der Schritt (b) durch Betreiben desselben ausgeführt wird.

8. Verfahren nach Anspruch 7 mit den Schritten des Durchscannens des einfallenden Diagnosestrahls über den Hintergrund auf mehrere geometrische Einfallspunkte und des Abbildens des Ausmaßes der Änderung des Polarisationszustands des zurückkehrenden Diagnosestrahls als Funktion der geometrischen Einfallspunkte.

9. Verfahren nach Anspruch 8, bei dem mindestens einer der Diagnosestrahlen hinsichtlich seines Polarisationszustands moduliert wird, um eine Abfolge modulierter Polarisationszustände zu erzeugen, wobei für jeden derartig modulierten Polarisationszustand des mindestens einen der Diagnosestrahlen der zurückkehrende Diagnosestrahl hinsichtlich jedes der beleuchteten Punkte des Hintergrunds analysiert wird, um eine Polarisationsangabe für jeden der beleuchteten Punkte herzuleiten, und die Polarisationsangaben für alle beleuchteten Punkte des Hintergrunds für jeden der modulierten Polarisationszustände als für einen modulierten Polarisationszustand spezifische Bilder in einem Computerspeicher (66) abgespeichert werden, mit dem Schritt des Berechnens des Ausmaßes der Änderung des Polarisationszustands des zurückkehrenden Diagnosestrahls aus den im Computerspeicher abgespeicherten für den modulierten Polarisationszustand spezifischen Polarisationsbildern für jeden beleuchteten Punkt des Hintergrunds, und mit dem Schritt des Abbildens des Ausmaßes der Änderung des Polarisationszustands des zurückkehrenden Diagnosestrahls als Funktion der beleuchteten Punkte des Hintergrunds.

10. Verfahren nach einem der vorstehenden Ansprüche, bei dem die polarisationsempfindliche Erfassungsvorrichtung einen Erfassungsscanner (58) aufweist, der den zurückkehrenden Diagnosestrahl sequenziell über mehrere Polarisatoren (62) mit verschiedenen Polarisationszuständen scannt, und mit den Schritten einer sequenziellen Intensitätsanalyse des zurückkehrenden Diagnosestrahls, nachdem er sequenziell über jeden der mehreren Polarisatoren (62) gescannt wurde, um für jeden der Polarisatoren einen polarisatorspezifischen Intensitätswert zu erzeugen, aus dem der Polarisationszustand des zurückkehrenden Diagnosestrahls berechnet werden kann.

11. Verfahren nach Anspruch 10, bei dem der einfallende Diagnosestrahl den Hintergrund beleuchtet, um mehrere Beleuchtungspunkte zu erzeugen, und der zurückkehrende Diagnosestrahl von jedem beleuchteten Punkt des Hintergrunds mittels eines Polarisators der mehreren Polarisatoren (62) gesammelt wird, was zu einem polarisatorspezifischen Polarisationsbild führt, und das polarisatorspezifische Polarisationsbild in einen Computerspeicher (66) eingespeichert wird, und mit dem Schritt des Wiederholens der unmittelbar zuvor angegebenen Prozeduren des Beleuchtens des Hintergrunds, des Sammelns des zurückkehrenden Diagnosestrahls und des Speicherns des polarisatorspezifischen Polarisationsbilds für jeden Polarisator der mehreren Polarisatoren (62), und mit dem Schritt des Abbildens des Ausmaßes der Änderung des Polarisationszustands des zurückkehrenden Diagnosestrahls für jeden Bildpunkt der im Computerspeicher gespeicherten Polarisationsbilder.

12. Verfahren nach Anspruch 1, mit einem Diagnosescanner (58) zum sequenziellen Scannen des einfallenden Diagnosestrahls über den Hintergrund auf mehrere geometrische Einfallspunkte auf dem Augenhintergrund, und mit dem Schritt des Abbildens des Ausmaßes der Änderung des Polarisationszustands des zurückkehrenden Diagnosestrahls als Funktion der auf dem Hintergrund durchgescannten geometrischen Einfallspunkte.

13. Verfahren nach Anspruch 1, bei dem die Nervenfaserschicht der Retina Doppelbrechung zeigt und sie über eine anteriore und eine posteriore Fläche verfügt, und es zum Schritt (a) gehört, das einfallende Licht im Wesentlichen auf eine Retinaschicht zu fokussieren, die im Wesentlichen posterior in Bezug auf die posteriore Fläche der Nervenfaserschicht liegt, und es zum Schritt (b) gehört, das Ausmaß der Änderung des Polarisationszustands des zurückkehrenden Diagnosestrahls zu erfassen, zu der es durch die Doppelbrechung der Nervenfaserschicht der Retina kommt, und mit dem Schritt des Umsetzens des Ausmaßes der Änderung des Polarisationszustands des zurückkehrenden Diagnosestrahls in einen Messwert für die Dicke der Nervenfaserschicht, und mit dem Schritt des Anzeigens des Messwerts für die Dicke der Nervenfaserschicht als Bild, das die Helligkeit mit der Dicke der Nervenfaserschicht der Retina korreliert, um ein Kartenbild für die Dicke der Nervenfaserschicht zu definieren.

14. Verfahren nach Anspruch 1, bei dem die Nervenfaserschicht der Retina über eine anteriore Fläche verfügt und zum Verfahren der Schritt des brennpunktmäßigen Durchscannens des einfallenden Diagnosestrahls über die anteriore Fläche in mindestens einer Brennebene mittels eines Diagnosescanners sowie der Schritt des Erzeugens einer Topografiekarte der anterioren Fläche durch Abbilden konfokal erfasster Scanpunkte, mit Reflexion am Hintergrund, wobei sich im reflektierten Licht minimale Nacheilung zeigt, gehören.

15. Verfahren nach Anspruch 14, bei dem es zum genannten Erzeugungsschritt gehört, Licht mit Polarisationsverschiebung dadurch auszufiltern, dass der zurückkehrende Diagnosestrahl durch ein Polarisationsfilter (30) mit einer wirksamen Polarisationsachse parallel zur Polarisationsachse des einfallenden Diagnosestrahls geschickt wird.

16. Verfahren nach Anspruch 1, bei dem der einfallende Diagnosestrahl eine erste Polarisationsachse aufweist und der zurückkehrende Diagnosestrahl eine zweite Polarisationsachse aufweist und es zum Schritt (a) gehört, die Polarisationsachse mindestens eines der Diagnosestrahlen zu verdrehen, um mehrere verschiedene sequenzielle Polarisationszustand-Beziehungen zwischen den Diagnosestrahlen und der doppelbrechenden Nervenfaserschicht der Retina zu erzeugen.

17. Verfahren nach Anspruch 16, mit den Schritten des Analysierens des reflektierten Lichts und des Erzeugens einer Intensitätskarte für die Nervenfaserschicht der Retina, die mit dem Maximalwert der Nacheilung korreliert, wie sie über die Drehung der Polarisationsachse für mindestens einen der Diagnosestrahlen für jeden beleuchteten Punkt auf der Nervenfaserschicht der Retina erzielt wird, mit einem entsprechenden Lichtintensitätswert auf der Karte.

18. Verfahren nach Anspruch 17, bei dem der Hintergrund eine Sehnervpapille mit einem Zentrum enthält und das Verfahren die Schritte des Analysierens des an Hintergrundorten entlang mindestens einer Linie enthält, die durch das genannte Zentrum der genannten Sehnervpapille läuft und unter einem festen Winkel zur sich drehenden Polarisationsachse eines der Diagnosestrahlen ausgerichtet ist.

19. Verfahren nach Anspruch 16, 17 oder 18, mit dem Schritt des Durchscannens des einfallenden Diagnosestrahls über den Hintergrund.

20. Verfahren nach Anspruch 1, bei dem die Nervenfaserschicht der Retina aus Nervenfasern besteht, die in örtlichen Bereichen über sie hinweg im Wesentlichen örtliche Parallelität zeigen, wobei diese Nervenfaserschicht der Retina auch Doppelbrechung mit einer optischen Achse im Wesentlichen parallel zur Ausrichtung der Nervenfasern zeigt, wobei es zum Schritt (a) gehört, den Hintergrund so zu beleuchten, dass zumindest einige der örtlichen Bereiche der Nervenfaserschicht beleuchtet werden, und es zum Schritt (b) gehört, die optische Achse der Nervenfaserschicht in jedem der örtlichen Bereiche zu bestimmen, und mit dem Schritt des Herleitens der Ausrichtung der Nervenfasern aus der optischen Achse in mindestens einigen der örtlichen Bereiche.

21. Verfahren nach Anspruch 20, bei dem der einfallende Diagnosestrahl eine erste Polarisationsachse aufweist und es zum Schritt (a) gehört, die Polarisationsachse mindestens eines der Diagnosestrahlen über im Wesentlichen 90° zu verdrehen, um mehrere Orientierungszustände der Polarisationsachse zu erzeugen, und es zum Schritt (b) gehört, das Ausmaß der Änderung des Polarisationszustands des zurückkehrenden Diagnosestrahls als Funktion des Rotationswinkels der Orientierungszustände der Polarisationsachse in jedem der örtlichen Bereiche der Nervenfaserschicht in jedem der örtlichen Bereiche dadurch zu bestimmen, dass der Rotationswinkel desjenigen Orientierungszustands der Polarisationsachse identifiziert wird, der das minimale Ausmaß der Änderung des Polarisationszustands des zurückkehrenden Diagnosestrahls erzeugt.

22. Vorrichtung zum Erkennen von Augenerkrankungen mit:
(a) einer Quelle (26) für polarisiertes Licht zum Erzeugen eines einfallenden Diagnosestrahls (48) mit bekanntem Polarisationszustand;
(b) einer Durchstrahleinrichtung (42, 44) zum Durchstrahlen des einfallenden Diagnosestrahls durch die Pupille hindurch in ein Auge, wobei er als zurückkehrender Diagnosestrahl (50) im Inneren des Auges reflektiert wird;
(c) einer optischen Sammeleinrichtung (40) zum Sammeln des zurückkehrenden Diagnosestrahls und zum Durchstrahlen desselben zu einer polarisationsempfindlichen Erfassungsvorrichtung (24);
(d) einer polarisationsempfindlichen Erfassungsvorrichtung (24) zum Sammeln von Information zum Polarisationszustand des zurückkehrenden Diagnosestrahls und zum Umwandeln derselben in ein elektrisches Signal in solcher Weise, dass die Intensität desselben eine Funktion des Polarisationszustands des zurückkehrenden Diagnosestrahls ist;
(e) einem Hornhaut-Polarisationskompensator mit einem variablen Verzögerer, durch den ein Kompensationsstrahl polarisierten Lichts geleitet wird und durch die Hornhaut auf eine Fläche der Augenlinse fokussiert wird, der an der Linsenfläche reflektierte Kompensationsstrahl erneut durch den variablen Verzögerer geführt wird, und einer Kompensator-Überwachungseinrichtung, die so betreibbar ist, dass das Ausmaß der kombinierten Polarisationsanderung des Kompensationsstrahls auf Grund seines doppelten Durchlaufs durch den Verzögerer und das anteriore Augensegment ermittelt wird, um dadurch die Polarisation mindestens eines Diagnosestrahlen so zu modifizieren, dass die kombinierten Polarisationseffekte auf die Diagnosestrahlen sowohl vom anterioren Augensegment als auch vom Hornhaut-Polarisationskompensator bekannt sind, was eine genaue Erfassung jeder Änderung des Polarisationszustands des zurückkehrenden Diagnosestrahls erlaubt, zu der es durch die Polarisationseigenschaften des posterioren Augenabschnitts kommt.

23. Vorrichtung nach Anspruch 21, mit einer Einrichtung (34-42), die so betreibbar ist, dass sie die Polarisation mindestens eines der Diagnosestrahlen moduliert.

24. Vorrichtung nach Anspruch 21 oder 22, bei der die Durchstrahleinrichtung (42, 44) so betreibbar ist, dass sie den einfallenden Diagnosestrahl (48) auf einen Brennpunkt im Auge fokussiert, und mit einer Lochblende, die konjugiert zum Brennpunkt vor der polarisationsempfindlichen Erfassungsvorrichtung positioniert ist, um den Detektor so einzuschränken, dass er nur Licht erfasst, das von Objekten im Brennpunkt aus dem einfallenden Diagnosestrahl reflektiert wurde.

25. Vorrichtung nach Anspruch 21 oder 23, bei der die Durchstrahleinrichtung einen Diagnosescanner (58) aufweist, der so betreibbar ist, dass er den einfallenden Diagnosestrahl über den Augenhintergrund scannt.

26. Vorrichtung nach Anspruch 24, bei der die Durchstrahleinrichtung eine Fokussiereinrichtung aufweist, die so betreibbar ist, dass sie den Brennpunkt des einfallenden Diagnosestrahls ändert.

27. Vorrichtung nach Anspruch 21, bei der die polarisationsempfindliche Erfassungsvorrichtung ein Ellipsometer (24) aufweist.

28. Vorrichtung nach Anspruch 21, 24 oder 25, bei der die polarisationsempfindliche Erfassungsvorrichtung mindestens zwei Detektoren (24, 46, 64) aufweist, die so betreibbar sind, dass sie im Wesentlichen mindestens zwei verschiedene Polarisationskomponenten des zurückkehrenden Strahls erfassen.

29. Vorrichtung nach den Ansprüchen 21, 24 oder 25, bei der die polarisationsempfindliche Vorrichtung ein Array von Polarisatoren (62) mit verschiedenen vorbestimmten Polarisationszuständen, eine Ablenkvorrichtung (60) zum sequenziellen Durchscannen des zurückkehrenden Diagnosestrahls auf die Polarisatoren (62), eine Sammeleinrichtung (64) zum sequenziellen Empfangen von Licht von den Polarisatoren, mindestens einen Detektor, der so betreibbar ist, dass er Licht von der Sammeleinrichtung empfängt und die Intensität des zurückkehrenden Diagnosestrahls in elektrische Signale mit einer Intensität umsetzt, die der Polarisation des Lichts von den Polarisatoren entspricht, aufweist.

30. Polarisationskompensator für das anteriore Augensegment zur Verwendung bei einem Diagnosesystem unter Verwendung von polarisiertem Licht bei der Diagnose von Augenerkrankungen, mit:
(a) einer Lichtquelle (26) zum Erzeugen eines einfallenden Kompensationsstrahls (48) in einem bekannten Polarisationszustand;
(b) einer Durchstrahleinrichtung (40, 42) zum Lenken des einfallenden Kompensationsstrahls (48) durch das anteriore Augensegment, damit er durch Bereiche des Auges reflektiert wird, die stärker anterior als die Hornhaut liegen, um einen reflektierten Kompensationsstrahl (50) zu bilden, der das Auge entlang einem optischen Pfad verlässt, der im Wesentlichen mit dem einfallenden Diagnosestrahl ausgerichtet ist;
(c) einem variablen Verzögerer (44), der optisch in den optischen Pfad eingefügt ist; und
(d) einer Kompensator-Überwachungseinrichtung, die so betreibbar ist, dass sie das Ausmaß der kombinierten Polarisationsverschiebung gegenüber dem einfallenden Kompensationsstrahl ermittelt, wenn dieser zweimal durch den variablen Verzögerer (44) und das anteriore Augensegment läuft, um den variablen Verzögerer (44) als reflektierter Kompensationsstrahl (50) zu verlassen, so dass jegliche Änderung des Polarisationszustands des reflektierten Strahls gegenüber dem Polarisationszustand des einfallenden Strahls, wie durch das anteriore Augensegment hervorgerufen, durch Einstellen des variablen Verzögerers neutralisiert werden kann.

31. Kompensator nach Anspruch 30, bei dem der einfallende Kompensationsstrahl (50) dadurch linear polarisiert wird, dass er durch einen polarisierenden Strahlteiler (30) von einer ersten Seite desselben hindurchgestrahlt wird, und der reflektierte Strahl an der zweiten Seite des polarisierenden Strahlteilers (30) reflektiert wird und auf einen Fotodetektor (46) fokussiert wird, und mit einem Viertelwellenverzögerer (36) im optischen Pfad in solcher Weise, dass dann, wenn der variable Verzögerer (44) korrekt eingestellt ist, der Fotodetektor (46) die maximale Intensität erfasst, wenn der variable Verzögerer (44) die Polarisation des anterioren Augensegment kompensiert hat.

32. Kompensator nach Anspruch 30 oder 31, mit einem netzförmigen Beugungsgitter (38), das im einfallenden Kompensationsstrahl angeordnet ist, um diesen in mehrere Unterstrahlen aufzuteilen.

33. Kompensator nach Anspruch 30, 31 oder 32, mit einer polarisationsempfindlichen Vorrichtung mit einem Ellipsometer (24), das so betreibbar ist, dass es einen einfallenden Diagnosestrahl (48) entlang einem Pfad durch den variablen Verzögerer (44) emittiert, der als zurückkehrender Diagnosestrahl (50) reflektiert wird, wobei die Diagnosestrahlen und die Kompensationsstrahlen im Wesentlichen übereinstimmende Pfade durch den variablen Verzögerer (44) und das anteriore Augensegment aufweisen, so dass der zurückkehrende Diagnosestrahl im Polarisationszustand automatisch so eingestellt wird, dass jegliche Änderung des Polarisationszustands kompensiert wird, die durch den anterioren Augenabschnitt hervorgerufen wird.

34. Kompensator nach Anspruch 33, bei dem die polarisationsempfindliche Vorrichtung (64) eine Lichtquelle zum Erzeugen eines polarisierten Strahls und eine Einrichtung zum Scannen desselben über die Nervenfaserschicht der Retina des Auges aufweist, wodurch es zur Reflexion eines zurückkehrenden Diagnosestrahls kommt, und mit einem Array von Polarisatoren (62) mit fortschreitenden Polarisationsgraden, die durch den zurückkehrenden Diagnosestrahl (50) sequenziell beleuchtet werden, und einer Einrichtung zum Lenken des zurückkehrenden Diagnosestrahls, nachdem er die Polarisatoren durchlaufen hat, zur polarisationsempfindlichen Erfassungsvorrichtung (64), um den Polarisationszustand zu erfassen.

35. Kompensator nach Anspruch 30, mit einer polarisationsempfindlichen Vorrichtung mit einer Tomografiesonde mit einem dreidimensionalen Scanner (59) und einem Analysator und einem Detektor zum Herausfiltern von zurückkehrendem Licht, das an der Nervenfaserschicht reflektiert wurde und dessen Polaristation geändert wurde, und mit einem Analysator zum Analysieren des zurückkehrenden Lichts, dessen Polarisation nicht geändert wurde, und einer Einrichtung (63), die so betreibbar ist, dass sie eine Topografiekarte der Nervenfaserschicht auf Grundlage einer Analyse geometrischer Punkte derselben, die kein in der Polarisation geändertes zurückkehrendes Licht erzeugen, anzeigt.

## Revendications

1. Procédé d'examen des propriétés de polarisation du fond de l'oeil tout en corrigeant sensiblement les effets de polarisation du segment antérieur de l'oeil, comprenant les étapes suivantes:
(a) d'éclairement du fond oculaire comprenant la couche de fibre nerveuse rétinienne à travers la pupille et le segment antérieur comprenant la cornée et le cristallin, avec de la lumière incidente au moins partiellement polarisée en un faisceau de diagnostic incident (48) d'un état de polarisation connu ;
(b) de détection de la lumière réfléchie par le fond oculaire à partir du faisceau de diagnostic incident, en tant que faisceau de diagnostic renvoyé (50) avec un dispositif de détection sensible à la polarisation ; et
(c) de correction de manière sensible des effets de polarisation du segment antérieur de l'oeil à travers lequel passe ledit faisceau de diagnostic (48, 50) en utilisant un élément de correction de polarisation de segment antérieur d'oeil comprenant un élément à retard variable à travers lequel un faisceau de correction de lumière polarisée est transmis et concentré à travers la cornée sur une surface du cristallin de l'oeil, le faisceau de correction réfléchi sur ladite surface de cristallin étant de nouveau transmis à travers ledit élément à retard variable, et un moyen de surveillance d'élément de correction pouvant être activé afin de déterminer le niveau de variation de polarisation cumulé du faisceau de correction du fait de son double passage à travers l'élément à retard et le segment antérieur de l'oeil, de telle sorte que l'état de polarisation dudit faisceau de diagnostic renvoyé (50) peut être analysé et comparé audit état de polarisation connu du faisceau de diagnostic incident afin de déterminer le niveau d'altération de l'état de polarisation dudit faisceau de diagnostic renvoyé (50) sur la base de l'effet de polarisation global du fond oculaire, et de détermination dudit effet de polarisation global du fond oculaire à partir dudit niveau d'altération, à partir duquel la présence et la progression de certaines maladies des yeux peut être déduite.

2. Procédé selon la revendication 1, dans lequel l'étape (c) est réalisée en utilisant un élément de correction de polarisation de segment d'oeil antérieur interposé sur le trajet de propagation d'au moins l'un desdits faisceaux et qui constitue ledit élément à retard variable (44).

3. Procédé selon la revendication 2, dans lequel l'étape (c) comprend le réglage dudit élément de retard variable (44) de manière à obtenir une valeur connue du retard global cumulé induit sur la lumière passant à travers ledit élément à retard et le segment antérieur de l'oeil.

4. Procédé selon la revendication 3, dans lequel l'étape (c) est exécutée en réglant ledit élément à retard variable (44) de telle sorte que le retard global cumulé dudit élément à retard variable et du segment intérieur de l'oeil soit sensiblement nul afin que le retard du faisceau de diagnostic renvoyé détecté dans l'étape (b) indique sensiblement de manière aussi précise que possible, le retard provoqué par le fond oculaire indépendamment de tout retard du segment antérieur de l'oeil.

5. Procédé selon la revendication 4, dans lequel l'étape (c) est réalisée en concentrant un faisceau de correction incident (32) sur une surface du cristallin de l'oeil produisant un faisceau de correction réfléchi, en faisant passer chacun desdits faisceaux de correction à travers ledit élément à retard variable (44) et en réglant ledit élément à retard variable (44) jusqu'à ce que les faisceaux de correction du côté de l'élément à retard variable distant par rapport à l'oeil soient tous les deux dans des états de polarisation sensiblement identiques, à l'exception d'un déphasage de 180° provoqué par la réflexion sur la surface du cristallin de l'oeil.

6. Procédé selon la revendication 5, dans lequel ledit faisceau de correction incident est produit par laser et polarisé linéairement avec un premier axe de polarisation et transmis à travers un élément à retard en quart d'onde (36), ledit faisceau de correction réfléchi revient à travers ledit élément à retard en quart d'onde (36) pour se polariser linéairement suivant un second axe de polarisation orthogonal par rapport audit premier axe de polarisation en l'absence des effets de polarisation du segment intérieur de l'oeil et de l'élément à retard variable, et ledit élément à retard variable (44) est réglé de telle sorte que l'état de polarisation du faisceau de correction réfléchi après passage à travers l'élément à retard en quart d'onde (36) ressemble de manière aussi proche que possible à un état de polarisation linéaire avec un axe de polarisation orthogonal par rapport audit premier axe de polarisation.

7. Procédé selon l'une quelconque des revendications précédentes, dans lequel ledit dispositif de détection sensible à la polarisation de l'étape (b) est un ellipsomètre (24) et l'étape (b) est exécutée en activant ce dernier.

8. Procédé selon la revendication 7, comprenant, en outre, les étapes de balayage dudit faisceau de diagnostic incident à travers ledit fond oculaire sur une pluralité de points d'incidence géométriques et de localisation du niveau d'altération de l'état de polarisation dudit faisceau de diagnostic renvoyé en fonction desdits points d'incidence géométriques.

9. Procédé selon la revendication 8, dans lequel l'état de polarisation d'au moins l'un desdits faisceaux de diagnostic est modulé afin de produire une séquence d'états de polarisation modulés, et pour chacun de tels états de polarisation modulés dudit au moins un desdits faisceaux de diagnostic ledit faisceau de diagnostic renvoyé est analysé pour chacun desdits points éclairés du fond oculaire afin de déduire un indicateur de polarisation pour chacun desdits points, et les indicateurs de polarisation pour tous les points éclairés du fond oculaire pour chacun desdits états de polarisation modulés sont mémorisés dans une mémoire d'ordinateur (66) en tant qu'images spécifiques de l'état de polarisation modulé, et comprenant l'étape de calcul du niveau d'altération de l'état de polarisation dudit faisceau de diagnostic renvoyé à partir des images spécifiques de l'état de polarisation modulé mémorisées dans la mémoire d'ordinateur pour chaque point éclairé du fond oculaire, et comprenant l'étape de localisation desdits niveaux d'altération de l'état de polarisation dudit faisceau de diagnostic renvoyé en fonction des points éclairés dudit fond oculaire.

10. Procédé selon l'une quelconque des revendications précédentes, dans lequel ledit dispositif de détection sensible à la polarisation comprend un dispositif de scrutation de détection (58) qui scrute ledit faisceau de diagnostic renvoyé de manière séquentielle à travers une pluralité d'éléments polarisants (62) d'états de polarisation différents et comprenant les étapes d'analyse séquentielle de l'intensité dudit faisceau de diagnostic renvoyé après qu'il a été scruté de manière séquentielle à travers chacun de ladite pluralité d'éléments polarisants (62) afin de produire une valeur d'intensité spécifique de l'élément polarisant pour chacun desdits éléments polarisants à partir de laquelle l'état de polarisation dudit faisceau de diagnostic renvoyé peut être calculé.

11. Procédé selon la revendication 10, dans lequel ledit faisceau de diagnostic incident éclaire ledit fond oculaire afin de produire une pluralité de points éclairés, et ledit faisceau de diagnostic renvoyé à partir de chaque point éclairé dudit fond oculaire est collecté à travers un élément polarisant de ladite pluralité d'éléments polarisants (62) conduisant à une image de polarisation spécifique de l'élément polarisant, et ladite image de polarisation spécifique de l'élément polarisant est mémorisée dans une mémoire d'ordinateur (66), et comprenant l'étape de répétition des procédures citées immédiatement précédemment d'éclairement dudit fond oculaire, de collecte dudit faisceau de diagnostic renvoyé et de mémorisation de ladite image de polarisation spécifique de l'élément polarisant, pour chaque élément polarisant de ladite pluralité d'éléments polarisants (62), et comprenant l'étape de localisation du niveau d'altération de l'état de polarisation dudit faisceau de diagnostic renvoyé pour chaque point d'image desdites images de polarisation mémorisées dans ladite mémoire d'ordinateur.

12. Procédé selon la revendication 1, et comprenant un dispositif de scrutation de diagnostic (58) scrutant le faisceau de diagnostic incident de manière séquentielle à travers ledit fond oculaire à une pluralité de points géométriques d'incidence sur ledit fond oculaire et comprenant l'étape de localisation du niveau d'altération de l'état de polarisation dudit faisceau de diagnostic renvoyé en fonction desdits points géométriques d'incidence scrutés sur ledit fond oculaire.

13. Procédé selon la revendication 1, dans lequel ladite couche de fibre nerveuse rétinienne présente une certaine biréfringence et présente une surface antérieure et une surface postérieure, et l'étape (a) comprend la focalisation poussée de ladite lumière incidente sur une couche rétinienne sensiblement postérieure à ladite surface postérieure de la couche de fibre nerveuse, et l'étape (b) comprend la détection du niveau d'altération de l'état de polarisation dudit faisceau de diagnostic renvoyé provoquée par la biréfringence de la couche de fibre nerveuse rétinienne et comprenant l'étape de transformation dudit niveau d'altération de l'état de polarisation dudit faisceau de diagnostic renvoyé en une lecture d'épaisseur de couche de fibre nerveuse et l'étape d'affichage de ladite lecture d'épaisseur de couche de fibre nerveuse sous la forme d'une image établissant une corrélation entre la luminosité et l'épaisseur de ladite couche de fibre nerveuse rétinienne afin de définir une image d'épaisseur de couche de fibre nerveuse localisée.

14. Procédé selon la revendication 1, dans lequel ladite couche de fibre nerveuse rétinienne présente une surface antérieure, le procédé comprenant l'étape de balayage focalisé dudit faisceau de diagnostic incident à travers ladite surface antérieure sur au moins un plan focal avec un dispositif de scrutation de diagnostic, et l'étape de création d'une carte topographique de ladite surface antérieure en localisant, par balayage et détection à partir d'un foyer commun, de points réfléchis à partir du fond oculaire qui présentent un retard minimum sur la lumière réfléchie.

15. Procédé selon la revendication 14, dans lequel ladite étape de création comprend le filtrage de la lumière à décalage de polarisation en faisant passer ledit faisceau de diagnostic renvoyé à travers un filtre polarisé (30) présentant un axe de polarisation efficace parallèle à l'axe de polarisation dudit faisceau de diagnostic incident.

16. Procédé selon la revendication 1, dans lequel ledit faisceau de diagnostic incident présente un premier axe de polarisation, et ledit faisceau de diagnostic renvoyé présente un second axe de polarisation, et l'étape (a) comprend la rotation de l'axe de polarisation d'au moins l'un desdits faisceaux de diagnostic afin de créer une pluralité de relations d'état de polarisation séquentielles différentes parmi lesdits faisceaux de diagnostic et la couche de fibre nerveuse rétinienne biréfringente.

17. Procédé selon la revendication 16, comprenant les étapes d'analyse de ladite lumière réfléchie et de réalisation d'une carte d'intensité de la couche de fibre nerveuse rétinienne qui effectue une corrélation entre le niveau maximum de retard obtenu au cours de la rotation de l'axe de polarisation d'au moins l'un desdits faisceaux de diagnostic sur chaque point éclairé sur la couche de fibre nerveuse rétinienne et un niveau d'intensité lumineuse correspondant sur ladite carte.

18. Procédé selon la revendication 17, dans lequel ledit fond oculaire contient une pupille optique avec un centre, le procédé comprenant les étapes d'analyse de ladite lumière réfléchie sur les emplacements du fond oculaire le long d'au moins une ligne qui passe à travers ledit centre de ladite pupille optique et est orientée suivant un angle fixe par rapport à l'axe de polarisation tournant de l'un desdits faisceaux de diagnostic.

19. Procédé selon la revendication 16, 17 ou 18, comprenant l'étape de balayage dudit faisceau de diagnostic incident à travers ledit fond oculaire.

20. Procédé selon la revendication 1, dans lequel ladite couche de fibre nerveuse rétinienne consiste en fibres nerveuses qui présentent un parallélisme local important sur des zones locales à travers celle-ci, et ladite couche de fibre nerveuse rétinienne présente une certaine biréfringence avec un axe optique sensiblement parallèle à l'orientation desdites fibres nerveuses, l'étape (a) comprenant l'éclairement dudit fond oculaire afin d'éclairer au moins certaines desdites zones locales de ladite couche de fibre nerveuse, et l'étape (b) comprenant la détermination de l'axe optique de ladite couche de fibre nerveuse dans chacune desdites zones locales et l'étape de déduction de l'orientation des fibres nerveuses par rapport audit axe optique dans lesdites au moins certaines zones desdites zones locales.

21. Procédé selon la revendication 20, dans lequel ledit faisceau de diagnostic incident présente un premier axe de polarisation, et ledit faisceau de diagnostic renvoyé présente un second axe de polarisation, et l'étape (a) comprend la rotation de l'axe de polarisation d'au moins l'un desdits faisceaux de diagnostic sur sensiblement 90° afin de produire une pluralité d'états d'orientation d'axe de polarisation et l'étape (c) comprend la détermination du niveau d'altération de l'état de polarisation dudit faisceau de diagnostic renvoyé en fonction de l'angle de rotation desdits états d'orientation d'axe de polarisation sur chacune desdites zones locales de ladite couche de fibre nerveuse sur chacune desdites zones locales en identifiant l'angle de rotation de l'état d'orientation d'axe de polarisation qui a produit le niveau minimum d'altération de l'état de polarisation dudit faisceau de diagnostic renvoyé.

22. Dispositif destiné à détecter des maladies des yeux, comprenant:
(a) une source de lumière polarisée (26) destinée à produire un faisceau de diagnostic incident (48) d'un état de polarisation connu ;
(b) un moyen d'émission (42, 44) destiné à émettre ledit faisceau de diagnostic incident dans un oeil à travers la pupille, dans lequel il est réfléchi de l'intérieur de l'oeil sous la forme d'un faisceau de diagnostic renvoyé (50) ;
(c) un moyen de collecte optique (40) destiné à collecter ledit faisceau de diagnostic renvoyé et à transmettre celui-ci à un dispositif de détection sensible à la polarisation (24) ;
(d) un dispositif de détection sensible à la polarisation (24) destiné à collecter et à transformer l'information se rapportant à l'état de polarisation dudit faisceau de diagnostic renvoyé en un signal électrique de telle sorte que l'intensité dudit signal électrique soit une fonction de l'état de polarisation du faisceau de diagnostic renvoyé ;
(e) un élément de correction de polarisation cornéenne comprenant un élément à retard variable à travers lequel un faisceau de correction de lumière polarisée est transmis et concentré à travers la cornée sur une surface du cristallin de l'oeil, le faisceau de correction réfléchi sur ladite surface de cristallin étant de nouveau transmis à travers ledit élément à retard variable, et un moyen de surveillance d'élément de correction pouvant être activé afin de déterminer le niveau de variation de polarisation cumulé du faisceau de correction du fait de son double passage à travers l'élément à retard et le segment antérieur de l'oeil, afin de modifier ainsi la polarisation d'au moins l'un desdits faisceaux de diagnostic de telle sorte que les effets de polarisation cumulés sur lesdits faisceaux de diagnostic à la fois du segment antérieur de l'oeil et de l'élément de correction de polarisation cornéenne sont connus, permettant une identification précise d'une altération quelconque de l'état de polarisation dudit faisceau de diagnostic provoquée par les propriétés de polarisation de la partie postérieure de l'oeil.

23. Dispositif selon la revendication 21, comprenant des moyens (34 à 42) pouvant être commandés de manière à moduler la polarisation d'au moins l'un desdits faisceaux de diagnostic.

24. Dispositif selon la revendication 21 ou 22, dans lequel ledit moyen de transmission (42, 44) peut être commandé de manière à concentrer ledit faisceau de diagnostic incident (48) sur un point focal de l'oeil et comprenant un diaphragme d'orifice ponctuel positionné de manière conjuguée par rapport audit point focal face audit dispositif de détection sensible à la polarisation afin de limiter ledit détecteur à détecter uniquement la lumière réfléchie à partir d'objets sur ledit point focal à partir dudit faisceau de diagnostic incident.

25. Dispositif selon la revendication 21 ou 23, dans lequel ledit moyen de transmission comprend un dispositif de scrutation de diagnostic (58) pouvant être commandé de manière à scruter ledit faisceau de diagnostic incident à travers ledit fond oculaire de l'oeil.

26. Dispositif selon la revendication 24, dans lequel ledit moyen de transmission comprend un moyen de concentration pouvant être commandé de manière à modifier le point focal dudit faisceau de diagnostic incident.

27. Dispositif selon la revendication 21, dans lequel ledit dispositif de détection sensible à la polarisation comprend un ellipsomètre (24).

28. Dispositif selon la revendication 21, 24 ou 25, dans lequel ledit dispositif de détection sensible à la polarisation comprend au moins deux détecteurs (24, 46, 64) pouvant être commandés de manière à détecter sensiblement au moins deux composantes de polarisation différentes dudit faisceau renvoyé.

29. Dispositif selon les revendications 21, 24 ou 25, dans lequel ledit dispositif sensible à la polarisation comprend un réseau d'éléments polarisants (62) d'états de polarisation prédéterminés différents, un dispositif réfléchissant (60) destiné à scruter ledit faisceau de diagnostic renvoyé de manière séquentielle sur lesdits éléments polarisants (62), un moyen de collecte (64) destiné à recevoir de manière séquentielle de la lumière provenant desdits éléments polarisants, au moins un détecteur pouvant être commandé de manière à recevoir de la lumière provenant dudit moyen de collecte et transformant l'intensité dudit faisceau de diagnostic renvoyé en signaux électriques d'intensité correspondant à la polarisation de la lumière provenant desdits éléments polarisants.

30. Dispositif de correction de polarisation de segment d'oeil antérieur destiné à une utilisation dans un dispositif de diagnostic utilisant la lumière polarisée pour le diagnostic de maladie des yeux, comprenant:
(a) une source de lumière (26) destiné à produire un faisceau de correction incident (48) dans un état de polarisation connu ;
(b) un moyen de transmission (40, 42) destiné à diriger ledit faisceau de correction incident (48) à travers le segment antérieur de l'oeil à réfléchir par des zones de l'oeil plus antérieures que la cornée, afin de former un faisceau de correction réfléchi (50) sortant de l'oeil le long d'un trajet optique sensiblement aligné avec le faisceau de correction incident ;
(c) un élément à retard variable (44) interposé optiquement sur ledit trajet optique ; et
(d) un moyen de surveillance d'élément de correction pouvant être commandé de manière à déterminer le niveau de décalage de polarisation cumulé par rapport audit faisceau de correction incident lorsqu'il passe deux fois à travers l'élément à retard variable (44) et le segment antérieur de l'oeil pour sortir dudit élément à retard variable (44) en tant que ledit faisceau de correction réfléchi (50), de telle sorte qu'aucune altération de l'état de polarisation dudit faisceau réfléchi par rapport à l'état de polarisation dudit faisceau incident provoqué par le segment antérieur de l'oeil peut être neutralisé en réglant ledit élément à retard variable.

31. Dispositif de correction selon la revendication 30, dans lequel ledit faisceau de correction incident (50) est polarisé linéairement en passant à travers un séparateur de faisceau polarisant (30) à partir d'un premier côté de celui-ci, et ledit faisceau réfléchi est réfléchi à partir du second côté dudit séparateur de faisceau polarisant (30) et concentré sur un photodétecteur (46) et comprenant un élément à retard en quart d'onde (36) sur ledit trajet optique de telle sorte que ledit élément à retard variable (44) est réglé correctement, ledit photodétecteur (46) détecte l'intensité maximum lorsque ledit élément à retard variable (44) a corrigé la polarisation dudit segment antérieur de l'oeil.

32. Dispositif de correction selon la revendication 30 ou 31, comprenant une grille de diffraction réticulée (38) disposée sur le faisceau de correction incident afin de fragmenter ledit faisceau de correction incident en une pluralité de faisceaux secondaires.

33. Dispositif de correction selon la revendication 30, 31 ou 32, comprenant un dispositif sensible à la polarisation comportant un ellipsomètre (24) pouvant être commandé de manière à émettre un faisceau de diagnostic incident (48) le long d'un trajet à travers ledit élément à retard variable (44) et qui est réfléchi sous la forme d'un faisceau de diagnostic renvoyé (50), lesdits faisceaux de diagnostic et lesdits faisceaux de correction présentant des trajets sensiblement coïncidents à travers l'élément à retard variable (44) et le segment antérieur de l'oeil, de telle sorte que l'état de polarisation du faisceau de diagnostic renvoyé est réglé de manière automatique afin de corriger toute altération de l'état de polarisation provoqué par la partie antérieure de l'oeil.

34. Dispositif de correction selon la revendication 33, dans lequel ledit dispositif sensible à la polarisation (64) comprend une source de lumière destinée à produire un faisceau polarisé et des moyens destinés à balayer ledit faisceau polarisé à travers la couche de fibre nerveuse rétinienne de l'oeil provoquant la réflexion d'un faisceau de diagnostic renvoyé, et comprenant un réseau d'éléments polarisants (62) de niveaux de polarisation progressifs qui sont éclairés de manière séquentielle par ledit faisceau de diagnostic renvoyé (50), et un moyen destiné à diriger ledit faisceau de diagnostic renvoyé, après être passé par lesdits éléments polarisants, vers ledit dispositif de détection sensible à la polarisation (64) afin de détecter l'état de polarisation.

35. Dispositif de correction selon la revendication 30, comprenant un dispositif sensible à la polarisation comportant une sonde de tomographie équipée d'un dispositif de scrutation tridimensionnel (59) et d'un analyseur et détecteur destiné à filtrer la lumière renvoyée réfléchie à partir de la couche de fibre nerveuse dont la polarisation a été altérée et d'un analyseur destiné à analyser la lumière renvoyée dont la polarisation n'a pas été altérée, et un moyen (63) pouvant être commandé de manière à afficher une carte topographique de la couche de fibre nerveuse sur la base d'une analyse des points géométriques de la couche de fibre nerveuse produisant une lumière renvoyée dont la polarisation n'a pas été altérée.
